(19) Europäisches Patentamt
European Patent Office
Office européen des brevets

(11) Veröffentlichungsnummer: **0 364 584 A1**

(12) # EUROPÄISCHE PATENTANMELDUNG veröffentlicht nach Art. 158 Abs. 3 EPÜ

(21) Anmeldenummer: 88906832.6

(51) Int. Cl.[5]: **A61K 31/44 , A61K 9/08**

(22) Anmeldetag: 27.04.88

(86) Internationale Anmeldenummer:
PCT/SU88/00103

(87) Internationale Veröffentlichungsnummer:
WO 89/10124 (02.11.89 89/26)

(43) Veröffentlichungstag der Anmeldung:
25.04.90 Patentblatt 90/17

(84) Benannte Vertragsstaaten:
AT BE CH DE FR GB IT LI NL SE

(71) Anmelder: KIEVSKY NAUCHNO-ISSLEDOVATELSKY INSTITUT FARMAKOLOGII I TOXIKOLOGII
ul. Ezhena Potie, 14
Kiev, 252057(SU)

(72) Erfinder: TRINUS, Fedor Petrovich
pr. Probedy, 12-13
Kiev, 252058(SU)
Erfinder: LOBODA, Julia Ivanovna
ul. Vladimiro-Lybedskaya, 22-37
Kiev, 252150(SU)
Erfinder: MUKHINA, Luiza Vladimirovna
ul. Sportivnaya, 10-18
Kiev, 252000(SU)
Erfinder: LITVINOV, Valentin Borisovich
ul. Erevanskaya, 13-1-94
Kiev, 252087(SU)
Erfinder: PORTNYAGINA, Vera Alexandrovna
ul. Bozhenko, 13 Kievskaya obl.
Boyarka, 255510(SU)
Erfinder: PANTELEIMONOV, Alexandr Gavrilovich
ul. Khorolskaya, 10-10
Kiev, 252090(SU)
Erfinder: FADEICHEVA, Alexandra Georgievna
ul. Karla Marxa, 5-11
Kiev, 252002(SU)
Erfinder: SOLUGUB, Petr Yakovlevich
pr. Probedy, 17-29
Kiev, 252055(SU)
Erfinder: PELIPAS, Svetlana Ivanovna
ul. Tychiny, 33-15
Kiev, 252097(SU)
Erfinder: MIRIAN, Natalya Ivanovna
ul. Chelyabinskaya, 3-125
Kiev, 252097(SU)
Erfinder: KONEV, Fedor Andreevich
ul. Engelsa, 10-11
Kharkov, 310023(SU)

(74) Vertreter: von Füner, Alexander, Dr. et al
Patentanwälte v. Füner, Ebbinghaus, Finck
Mariahilfplatz 2 & 3
D-8000 München 90(DE)

(54) ## ANTIDOT GEGEN VERGIFTUNG MIT PHOSPHORORGANISCHEN VERBINDUNGEN.

(57) Die vorliegende Erfindung bezieht sich auf die Medizin.
Das Antidot bei der Vergiftung mit phosphororganischen Verbindungen besteht aus dem Wirkstoff N-Allyl-2-pyridinaldoximbromid folgender Formel:

EP 0 364 584 A1

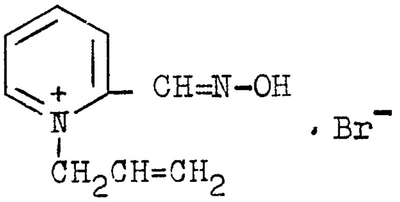

und einem pharmazeutischen Lösungsmittel.

# ANTIDOT GEGEN VERGIFTUNG MIT PHOSPHORORGANISCHEN VERBINDUNGEN

## Gebiet der Technik

Die vorliegende Erfindung bezieht sich auf die Medizin und betrifft insbesondere ein neues Antidot bei der Vergiftung mit phosphororganischen Verbindungen.

## Zugrundeliegender Stand der Technik

Zur Zeit ist ein Antidot bei der Vergiftung mit phosphororganischen Verbindungen TMB-4 (Trimethylen-1,3-bis-(4-oxyiminomethylpyridinium)bromid) bekannt (US, A, 3045025). Das genannte Präparat wird durch eine niedrige Fähigkeit gekennzeichnet, die hämatoenzephalische Barriere zu überwinden und das Zentralnervensystem zu beeinflussen, was dessen therapeutischer Effekt, besonders bei schweren Formen der Vergiftung mit phosphororganischen Verbindungen wesentlich herabsetzt.

## Offenbarung der Erfindung

Das beanspruchte Antidot ist neu und wurde in der Fachliteratur nicht beschrieben. Der Erfindung liegt die Aufgabe zugrunde, ein neues Antidot bei der Vergiftung mit phosphororganischen Verbindungen zu entwickeln, das eine zentrale und periphere Wirkung ausübt, eine hohe Wirksamkeit in schweren Fällen der Vergiftung und eine niedrige Toxizität besitzt.

Die Aufgabe wurde dadurch gelöst, daß das vorgeschlagene Antidot bei der Vergiftung mit phosphororganischen Verbindungen, das einen Wirkstoff und ein pharmazeutisches Lösungsmittel vorsieht, erfindungsgemäß als Wirkstoff N-Allyl-2-pyridinaldoximbromid folgender Formel enthält:

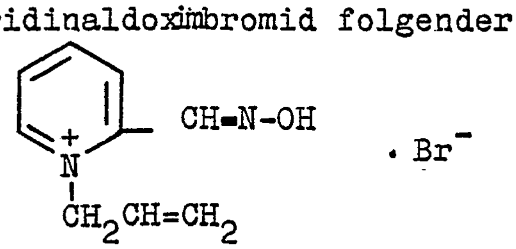

Das erfindungsgemäße Antidot wird in Form von Injektionslösungen angewendet. Erfindungsgemäß enthält das angebotene Antidot vorzugsweise den Wirkstoff in einer Menge von 7,5 Masse% und als pharmazeutisches Lösungsmittel

bidestilliertes Wasser.

Das erfindungsgemäße Antidot ist ein Cholinesterasereaktivator, der eine ausgeprägte therapeutische Wirkung bei akuten Vergiftungen mit phosphororganischen Verbindungen besitzt. Seine therapeutische Effektivität äußert sich sowohl in der peripheren als auch in der Zentralwirkung und wird durch eine schnelle Wiederherstellung des Bewußtseins der Kranken, die Verbesserung der bioelektrischen Hirnaktivität und die Wiederherstellung der neuromuskulären Leitfähigkeit gekennzeichnet.

Das erfindungsgemäße Arzneimittel dringt bei der intramuskulären Einführung (2 bis 5 min) in den Blutstrom ein, indem das Maximalniveau nach 20 bis 30 min erreicht wird. Das erfindungsgemäße Arzneimittel dringt in das Zentralnervensystem in viel höheren Mengen ein, als das bekannte Präparat TMB-4, und bleibt im Organismus eine längere Zeit.

Bei schweren Vergiftungen sichert das erfindungsgemäße Arzneimittel einen zuverlässigen therapeutischen Effekt.

Das erfindungsgemäße Arzneimittel übt in therapeutischen Dosen keinen Einfluß auf die Blutbildung, das kardiovaskuläre System, die Atmung, die Leber- und Nierenfunktion aus, wird von den Kranken bei einer dauernden Medikation gut vertragen.

## Beste Ausführungsform der Erfindung

Das erfindungsgemäße Antidot wurde im Tierversuch und in der Klinik an Menschen geprüft.

Im Versuch wurde die reaktivierende Wirksamkeit des erfindungsgemäßen Arzneimittels untersucht.

Die reaktivierende Wirksamkeit des erfindungsgemäßen Arzneimittels im Versuch wurde an der Cholinesterase des Grundgerüstes der Erythrozyten des menschlichen Bluts, die mit phosphororganischen Verbindungen (0,0-Dimethyl-(2,2,2-trichlor-I-oxyäthyl)phosphonat und 0,0-Dimethyl-S-(1,2-dikarbäthoxyäthyl)-dithiophosphat) inhibiert wurde, im Vergleich zum bekannten Präparat TMB-4 untersucht. Die Wiederherstellung der Fermentaktivität wurde bei einer

Temperatur von 25°C im Phosphatpuffer bei einem pH-Wert von 7,5 untersucht.

Die Hemmung der Cholinesteraseaktivität wurde durch die Zugabe der genannten Inhibitoren in einer Menge durchgeführt, die deren Hemmung um 70 bis 90% für 30 min sichert. Die Wiederherstellung des gehemmten Ferments mit dem erfindungsgemäßen Arzneimittel und TMB-4 wurde bei einer Konzentration von $1 \cdot 10^{-4}$ Mol/l durchgeführt. Die Aktivität des Ferments wurde nach der Methode der potentiometrischen Titration 0,5, 1 und 2 Stunden nach der Einwirkung mit phosphororganischen Verbindungen bestimmt.

Das Arzneimittel stellt die mit den genannten phosphororganischen Verbindungen gehemmte Azetylcholinesterase schneller als TMB-4 und bis zu einem höheren Niveau wiederher.

Die Untersuchungsergebnisse sind in der Tabelle 1 angeführt.

Tabelle 1

Reaktivierende Aktivität des erfindungsgemäßen Arzneimittels im Vergleich zu TMB-4 in bezug auf Azetylcholinesterase des menschlichen Bluts, die mit phosphororganischen Verbindungen inhibiert wurde

| Lfd. Nr. | Phosphororganische Verbindungen | Reaktivator | Aktivität der Azetylcholinesterase in % (Reaktivationszeit in Stunden) | | |
|---|---|---|---|---|---|
| | | | 0,5 | 1 | 2 |
| 1. | 0,0-Dimethyl-(2,2,2-trichlor-1-oxyäthyl) phosphonat | das erfindungsgemäße Arzneimittel | 53,6 | 72,2 | 72,3 |
| 2. | -"- | TMB-4 | 40,0 | 51,0 | 64,2 |
| 3. | 0,0-Dimethyl-S-(1,2-dikarbäthoxyäthyl)-dithiophosphat | das erfindungsgemäße Arzneimittel | 48,1 | 75,0 | 78,1 |
| 4. | -"- | TMB-4 | 27,4 | 43,0 | 52,9 |

Die reaktivierende Aktivität des erfindungsgemäßen Arzneimittels in vivo im Blut und Gehirn wurde an weißen Ratten untersucht, die mit den genannten phosphororganischen Verbindungen in der Dosis von $LD_{50}$ bei der enteralen Einführungsform vergiftet wurden. Die Untersuchungen wurden im Vergleich zum Präparat TMB-4 durchgeführt. Die Präparate wurden in einer Dosis von 10 mg/kg zusammen mit Atropin in einer Dosis von 5 mg/kg einmalig intramuskulär 2-3 min nach der Einführung der phosphororganischen Verbindungen verabreicht. Die Cholinesteraseaktivität im intakten Blut und Gehirn der dekapitierten Tiere wurde nach der Methode von Hestrin 0,5, 1 Stunde und 1, 3 und 5 Tagen nach der Vergiftung bestimmt. Parallel wurde zu der gleichen Frist die Aktivität der Cholinesterase im Blut und Gehirn der Kontrollrattengruppe untersucht, denen phosphororganischen Verbindungen, jedoch keine Arzneimittel eingeführt wurden.

Die Untersuchungen haben ergeben, daß die Ausnutzung des erfindungsgemäßen Arzneimittels in Kombination mit Atropin als Antidot bei der Vergiftung der Ratten mit phosphororganischen Verbindungen zu einer bedeutenden Wiederherstellung der Cholinesteraseaktivität im Blut und Gehirn beiträgt. Die Ergebnisse der Untersuchungen sind in Tabelle 2 dargestellt.

Auf Grund der durchgeführten Untersuchungen wurde festgestellt, daß das erfindungsgemäße Arzneimittel unter den Bedingungen der Vergiftung der Tiere mit phosphororganischen Verbindungen eine deutliche reaktivierende Wirkung auf die mit phosphororganischen Verbindungen inhibierte Cholinesterase des intakten Bluts und des Gehirns der Tiere ausübt und seiner spezifischen Wirkung nach das bekannte Präparat TMB-4 übersteigt.

Mit Hilfe der Methode von Askew (1956) wurde in der Dynamik der Gehalt des erfindungsgemäßen Arzneimittels im Blut und Gehirn der Tiere untersucht. Die Untersuchungen wurden im Vergleich zum bekannten Präparak TMB-4 vorgenommen. Die Geschwindigkeit des Eindringens beider Präparate ins Blut und deren Konzentration wurden bei der intramuskulären Einführung in Dosen von 20 und 50 mg/kg und der Gehirnspiegel - in einer Dosis von 50 mg/kg

bestimmt. Die quantitative Bestimmung der Präparate wurdde im Blut und im Gehirn zu verschiedenen Zeitabständen nach deren Einführung (5, 10, 20, 30 min und 1, 3 und 4 Stunden) durchgeführt.

Es wurde festgestellt, daß die Präparate schnell (nach 5 min) von der Einführungsstelle ins Blut eindringen, dabei wurde unabhängig von der Prüfdosis der Präparate deren Maximalgehalt im Blut 20 min nach der Einführung beobachtet, zwar erhöhte die Vergrößerung der Dosis der Präparate bis 50 mg/kg wesentlich deren Konzentrationsniveau während der ganzen Beobachtungsfrist.

Tabelle 2

Cholinesteraseaktivität im Blut und Gehirn weißer Ratten bei der Intoxikation mit phosphororganischen Verbindungen und der Behandlung mit dem erfindungsgemäßen Arzneimittel und TMB-4 in Kombination mit Atropin

| Lfd. Nr. | Phosphororganische Verbindung | Antidot+ Atropin | Aktivität der Azetylcholinesterase zu verschiedener Zeit nach der Einführung phosphororganischer Verbindungen (in % vom Ausgangsniveau) | | | | |
|---|---|---|---|---|---|---|---|
| | | | Blut | | | | |
| | | | 0,5 h | 1 h | 1 Tag | 3 Tage | 5 Tage |
| 1 | 2 | 3 | 4 | 5 | 6 | 7 | 8 |
| 1. | O,O-Dimethyl-(2,2,2-trichlor-1-oxyäthyl)-phosphonat | - | 14,6 | 19,4 | 24,5 | 42,2 | 54,2 |
| 2. | -"- | das erfindungsgemäße Präparat +Atropin | 59,0 | 48,9 | 56,7 | 82,9 | 85,0 |
| 3. | -"- | TMB-4 + Atropin | 29,9 | 39,7 | 54,9 | 59,3 | 64,6 |
| 4. | O,O-Dimethyl-(1,2-dikarbäthoxyäthyl)-dithiophosphat | -"- | 52,0 | 35,7 | 19,4 | 22,2 | 36,0 |
| 5. | -"- | das erfindungsgemäße Präparat+Atropin | 70,9 | 58,0 | 51,7 | 49,8 | 75,0 |
| 6. | -"- | TMB-4+Atropin | 67,7 | 50,6 | 37,2 | 55,3 | |

Fortsetzung der Tabelle 2

| Lfd. Nr. | Aktivität der Azetylcholinesterase zu verschiedener Zeit nach der Einführung phosphororganischer Verbindungen (in % vom Ausgangsniveau) | | | | |
|---|---|---|---|---|---|
| | Gehirn | | | | |
| | 0,5 h | I h | I Tag | 3 Tage | 5 Tage |
| I | 9 | IO | II | I2 | I3 |
| I. | 2I,2 | 32,I | 49,4 | 49,5 | 53,4 |
| 2. | 49,7 | 33,3 | 66,3 | 80,3 | 77,8 |
| 3. | 25,4 | 53,5 | 60,4 | 67,0 | 67,7 |
| 4. | 32,I | 22,4 | 22,4 | 3I,6 | 52,9 |
| 5. | 83,0 | 72,8 | 48,0 | 52,I | 7I,8 |
| 6. | 3I,6 | 2I,2 | 37,2 | 49,6 | 67,0 |

Die im Vergleich zu TMB-4 durchgeführten Untersuchungen haben ergeben, dass während der ersten Stunde der Blutgehalt und das Anwachsen der Konzentrationen beider Präparate gleich waren. Im weiteren jedoch (3 und 4 Stunden) war das Konzentrationsniveau des erfindungsgemässen Arzneimittels bedeutend (ungefähr um das Doppelte) höher als von TMB-4. Die Untersuchungsergebnisse sind in der Tabelle 3 angeführt.

Tabelle 3

Gehalt des erfindungsgemässen Arzneimittels und von TMB-4 im Blut und Gehirn der Ratten zu verschiedener Zeit nach der Einführung

| Lfd. Nr. | Arzneimittel | Dosis mg/kg | Konzentration (µg/g) 5 min | 10 min | 20 min | 30 min | I St. | 2 St. | 3 St. |
|---|---|---|---|---|---|---|---|---|---|
| | | Blut | | | | | | | |
| I. | Das erfindungsgemässe Präparat | 20 | 2,95 | 4,95 | 5,I6 | 4,7 | 4,4 | 2,3 | 0,92 |
| 2. | TMB-4 | 20 | 3,6 | 4,42 | 4,85 | 4,35 | 2,75 | 0,4I | - |
| 3. | Das erfindungsgemässe Präparat | 50 | I6,2 | I9,0 | I8,0 | I2,7 | IO,I | 4,6 | 3,6 |
| 4. | TMB-4 | 50 | I6,6 | 2I,I | I8,4 | I5,I | II,5 | 2,42 | I,95 |
| | | Gehirn | | | | | | | |
| 5. | Das erfindungsgemässe Präparat | 50 | 2,64 | 5,65 | 4,0 | 3,75 | 2,06 | I,I2 | 0,64 |
| 6. | TMB-4 | 50 | 0,86 | 0,98 | 2,32 | 2,28 | I,5 | 0,34 | - |

Nach den Ergebnissen der durchgeführten Untersuchungen wurde festgestellt, dass das erfindungsgemässe Arzneimittel bei der intramuskulären Einführung schnell ins Blut und Gehirn des Tieres eindringt und zum Unterschied von TMB-4 eine längere Zeit im Blutstrom zirkuliert und von 5 min an bis 4 Stunden Beobachtung im Gehirn in bedeutend grösseren Mengen enthalten ist.

Wie es festgestellt wurde, dringt das erfindungsgemässe Arzneimittel leicht durch die hämatoenzephalische Barriere bei der Einführung den intakten Tieren (Ratten) durch, wovon die Hemmung der Aktivität der Hirncholinesterase zeugt.

Die Hemmung der Cholinesterase geht in vitro um 50% im Gehirnhomogenat bei einer Konzentration des erfindungsgemässen Arzneimittels von $5,I \cdot IO^{-5}$ Mol/l vor sich.

Die Ergebnisse der Untersuchungen sind in der Tabelle 4 dargestellt.

Tabelle 4

Aktivität der Hirncholinesterase der Ratten eine Stunde nach der Einführung des erfindungsgemässen Arzneimittels

| Dosis des erfindungsgemässen Arzneimittels, mg/kg | Chloinesteraseaktivität, % | $ED_{50}$, mg/kg |
|---|---|---|
| 20 | 38,0±I,29 | |
| 35 | 25,6±0,89 | I,5,5±0,42 |
| 50 | I5,8±I,4 | |

Nach den histochemischen Methoden wurde der Einfluss des erfindungsgemässen Arzneimittels auf die Aktivität der Azetyl- und Butyrylcholinesterase des Rattengehirns untersucht und es wurde gezeigt, dass dieser von der Dosis des Arzneimittels und der Einführungszeit abhängt. So ruft das erfindungsgemässe Arzneimittel in einer Dosis von 5 mg/kg keine Abweichung der Fermentaktivität von der Norm hervor, und in einer Dosis von 20 mg/kg verändert es auch im wesentlichen die Aktivität der Azetylcholinesterase nicht, mit Ausnahme des Ferments in der weissen Substanz der Grosshirnhemisphären und in den Neuronen der

Formatio reticularis des Gehirmstammes, wo die Fermenthemmung (von 30 min bis 2 Stunden) mit darauffolgender unbedeutender Aktivierung im Laufe von 24 Stunden stattfindet. Das erfindungsgemässe Arzneimittel übt in einer Dosis von 50 mg/kg deutlicher ausgeprägte Veränderungen der Azetylcholinesteraseaktivität in den Neuronen des Schweifkerns, des Sehhügels, der knieförmigen Körper, der Formatio reticularis des Markhirns, in den eigenen Kernen und Purkinjeschen Zellen des Kleinhinrs, in der weissen Substanz der Grosshirnhemisphären aus. Die Fermenthemmung wird bis 2 Stunden lang beobachtet, nachher folgt eine unbedeutende Aktivierung der Azetylcholinesterase, die bis 24 Stunden aufrechterhalten bleibt. In den Neuronen der Grosshirnrinde wurden bei der Anwendung des erfindungsgemässen Arzneimittels in einer Dosis von 20 und 50 mg/kg keine Veränderungen der Aktivität der Azetylcholinesterase festgestellt.

In allen untersuchten Konzentrationen beeinflusst das erfindungsgemässe Arzneimittel die Aktivität der Butyrylcholinesterase in den Nervenkittzellen des Gehirns nicht. Jedoch wird in den Kapillaren aller Bildungen des Gehirns bis 2 Tage lang eine unbedeutende Aktivierung des Ferments nachgeweisen, die von der Dosis des erfindungsgemässen Arzneimittels abhängt (deutlicher ausgeprägt bei einer Dosis von 50 mg/kg). Es muss hervorgehoben werden, dass die beobachteten Veränderungen der Aktivität des Ferments von keinem deutlich ausgeprägten Charakter sind.

Histochemische Untersuchungen des Gehirns der Tiere (Ratten) zeigten eine allgemeine unbedeutende Kapillarhyperämie unter dem Einfluss des erfindungsgemässen Arzneimittels. Der Grad deren Ausgeprägtheit wird etwas höher und länger in den Gehirnstrukturen (3 Tage) bei einer Dosis des erfindungsgemässen Arzneimittels von 50 mg/kg aufrechterhalten, als bei Dosen von 5 und 20 mg/kg. Die Kapillarhyperämie wird durch keine Blutergüsse und Störungen des argyrophilen Gestells der Hirnblutgefässe begleitet. Die morphologische und histochemische Untersuchung des Rattengehirns, denen das erfindungsgemässe Arzneimittel in Dosen von 5, 20 und 50 mg/kg eingeführt wurde, gestattet, die erhaltenen Veränderungen als funktionelle einzuschätzen (die Schwankung der Aktivität der Azetylcholinesterase und der Butyrylcholinesterase, die papilläre Hyperämie), die von einer deutlichen Neurotropie des Arzneimittels zeugen.

Das erfindungsgemässe Arzneimittel übte keinen Einfluss auf das kardiovaskuläre System und die Atmung bei der subkutanen Einführung in hohen Dosen (I0 und 20 kg/kg) aus, und bei der intravenösen Einführung (I2 bis I5 mg/kg) setzte unbedeutend für eine kürzere Zeit den arteriellen Blutdruck herab. Das erfindungsgemässe Arzneimittel übte keine myotrope Wirkung aus.

Die therapeutische Effektivität des erfindungsgemässen Arzneimittels wurde an weissen Mäusen bei der peroralen Einführung der phosphororganischen Verbindungen in der Dosis $LD_{50}$ im Vergleich zu den aktivsten Arzneimitteln - Reaktivatoren der peripheren Wirkung TMB-4 und Toxogonin und zum Arzneimittel - Reaktivator vom zentralen Wirkungstyp Isonitrosin untersucht, das durch die hämatoenzephalische Barriere im Zentralnervensystem in höherem Masse als Toxogonin und TMB-4 eindringt.

Alle genannten Arzneimittel wurden den Tieren in Kombination mit Atropin intramuskulär 2 bis 3 min nach der Verabreichung der phosphororganischen Verbindungen eingeführt.

Die therapeutische Effektivität der Arzneimittel wurde nach der Grösse des Schutzindexes bewertet, der das Verhältnis der $LD_{50}$ der phosphororganischen Verbindungen unter den Bedingungen der Ausnutzung eines Antidots zur $LD_{50}$ der phosphororganischen Verbindungen ohne Ausnutzung des Antidots darstellt.

Die Untersuchungsergebnisse sind in der Tabelle 5 angeführt.

Tabelle 5

Therapeutische Effektivität der Arzneimittel in Kombination mit Atropin bei der Vergiftung weisser Ratten mit phosphororganischen Verbindungen in der Dosis $LD_{50}$

| Lfd. Nr. | Die phosphororganische Verbindung | Antidot | Schutzindex |
|---|---|---|---|
| I. | 0,0-Dimethyl-(2,2,2-trichlor-I-oxyäthyl)phosphonat | das erfindungsgemässe Arzneimittel (IO mg/kg) + Atropin (5 mg/kg) | 3,23 |
| 2. | -"- | TMB-4 (IO mg/kg) + Atropin (5 mg/kg) | 3,I2 |
| 3. | -"- | Toxogonin (IO mg/kg) + Atropin (5 mg/kg) | 2,67 |
| 4. | -"- | Isonitrosin (20 mg/kg) + Atropin (5 mg/kg) | 2,5 |
| 5. | 0,0-Dimethyl-S-(I,2-dikarbäthoxyäthyl)dithiophosphat | das erfindungsgemässe Arzneimittel (IO mg/kg) + Atropin (5 mg/kg) | 2,54 |
| 6. | -"- | TMB-4 (IO mg/kg) + Atropin (5 mg/kg) | 2,07 |
| 7. | -"- | Toxogonin (IO mg/kg) + Atropin (5 mg/kg) | I,46 |
| 8. | -"- | Isonitrosin (20 mg/kg) + Atropin (5 mg/kg) | I,3 |

Anhand der genannten Ergebnisse wurde festgestellt, dass die am deutlichsten ausgeprägte therapeutische Wirkung (nach dem Schutzindex zu urteilen) das erfindungsgemässe Arzneimittel ausübte.

Es wurde die Toxitität des erfindungsgemässen Arzneimittels untersucht.

Die Toxizität des erfindungsgemässen Arzneimittels bei der einmaligen Einführung (die akute Toxizität) wurde an geschlechtsreifen Tieren (an Mäusen, Ratten, Katzen, Hunden) bestimmt. Die Analyse der experimentellen Angaben für Mäuse und Ratten wurde nach der Methode der Probit-Analyse nach Litchfield und Wilcoxon, und für Hunde und Katzen nach der Methode der Anreicherung der

Frequenz nach Behrens durchgeführt.

Die Toxizitätsparameter $LD_{50}$ wurden für Mäuse bei der intraperitonealen und subkutanen Einführung, für Ratten bei der subkutanen, intramuskulären und enteralen Einführung, für Katzen bei der intramuskulären Einführung und für Hunde bei der subkutanen Einführung bestimmt.

Die erhaltenen Angaben sind in der Tabelle 6 angeführt.

Bei einer dauernden Anwendung übte das erfindungsgemässe Arzneimittel keinen merklichen Einfluss auf den Bestand des peripheren Bluts aus. Nur zu einer späteren Beobachtungsperiode (4 bis 9 Tage) wurde eine unbedeutende Erhöhung der Gesamtleukozytenzahl nachgewiesen. Am 3. Tag trat auch eine geringe Aktivierung der Blutcholinesterase auf, in den nachfolgenden Beobachtungstagen (4 bis 9 Tage) überstieg jedoch die Aktivität des Ferments das Ausgangsniveau nicht.

Tabelle 6

Toxizitätsparameter des erfindungsgemässen Arzneimittels für verschiedene Tierspezies

| Tierart | Einführungsform | $LD_{50}$ (mg/kg) und deren zuverlässige Grenzen P = 0,05 |
|---|---|---|
| Mäuse | intraperitoneal | 64 (33,6 - I2I,6) |
| | subkutan | II5 (99,I - I33,3) |
| Ratten | intraperitoneal | I35 (I05,4 - I72,8) |
| | subkutan | 200 (I37,9 - 280,0) |
| | enteral | 260 (2I4,0 - 3I4,6) |
| Hunde | subkutan | II2 |
| Katzen | intramuskulär | I26 |

Eine längere Einführung des erfindungsgemässen Arzneimittels veränderte nicht den Harnbestand, was wie das Ausbleiben der Nebenwirkung auf die Nieren bewertet werden kann.

Die morpholigischen Untersuchungen der inneren Organe der Versuchstiere legten keine pathologischen Veränderungen im Vergleich zur Kontrolle an den Tag.

Das erfindungsgemässe Arzneimittel wurde in der Klinik an Kranken mit akuten peroralen Vergiftungen mit verschiedenen phosphororganischen Insektiziden untersucht. Insgesamt wurden I83 Menschen behandelt, die in die Klinik gelangten. Daraus befanden sich 26 Menschen in einer leichten, 48 Kranke - in einer mässigen und 75 Kranke in einer schweren Vergiftungsstufe. Das Alter der Geschädigten schwankte von I5 bis 76 Jahren. Die Zeit des Behandlungsbeginns betrug I bis I2 Stunden nach der Einnahme des Insektizids und in 28 Fällen wurde die Behandlung vor der Hospitalisierung begonnen.

Die therapeutische Effektivität des erfindungsgemässen Arzneimittels wurde mit der ähnlichen Wirkung des wirksamsten bekannten Präparats TMB-4 verglichen (insgesamt 50 Menschen).

Das klinische Bild der akuten Vergiftung mit phosphororganischen Insektiziden beim Gelangen der Geschädigten in die Klinik war von einem deutlichen neorogenen Charakter. In allen Fällen hatten die Störungen seitens des Zentralnervensystems statt, die durch verschiedenartige Veränderungen der psychischen Aktivität der Kranken gekennzeichnet wurden: das frühere asthenischen Syndrom, psychomotorische Aufregung, komatöser Zustand. Es wurde das klinische Bild der Läsion des peripheren Nervensystems beobachtet (nikotinähnliche Wirkung des Insektizids äusserte sich in der Muskelschwäche, Myofibrillationen, peripheren Extremitätenlähmung). Die Störungen seitens der Atmung wurden durch die Bronchorrhoe sowie durch die Störungen nach dem Zentraltyp in Form von Rigidität des Brustkastens, dessen Lähmung, der Lähmung des Atmungszentrums gekennzeichnet. Seitens des kardiovaskulären Systems wurden ein früheres hypertonisches Syndrom, Bradykardie, Kollaps nachgewiesen. Die Störungen seitens des Magendarmkanals wurden in Form von Ubelkeit, Erbrechen, weheartigen Schmerzen im Magen, Diarrhoe verzeichnet.

Die Cholinesteraseaktivität des intakten Bluts bei den Kranken war um IO bis 90% herabgesetzt.

Das erfindungsgemässe Arzneimittel wurde in Kombination mit Atropin neben aktiven Methoden der Detoxikation

ausgenutzt, die die Giftentfernung aus dem Organismus bezweckten und das Durchwaschen des Magens, die Verordnung von Abführungsmitteln, Siphon- und Abführungsklystieren, die Beschleunigung der Diurese, aktive symptomatische und Erhaltungstherapie, allgemein stärkenden Behandlung, Massnahmen zur Korrektion der Störungen des säurig-alkalischen und wässrigen und Elektrolytzustands einschliessen.

Ausserdem wurde den Kranken mit schweren Vergiftungen nach Notwendigkeit die künstliche Lungenvetilation und die extrakorporale Hämosörption durchgeführt (als Sorptionsmittel wurde Aktivkohle ausgenutzt).

Das erfindungsgemässe Arzneimittel wurde in einer Einzeldosis von 0,075 g bei der intramuskulären Einführung in einem Abstand zwischen den Injektionen von I bis 3 Stunden innerhalb der ersten 24 Stunden Intoxikation neben der intensiven Erhaltungs-Atropinbehandlung verwendet. Die Gesamtdosis des erfindungsgemässen Arzneimittels pro eine Behandlungskur betrug 0,4 bis I,6 g.

Bei der Behandlung mit dem erfindungsgemässen Arzneimittel wurde seine ausgeprägte Zentralwirkung festgestellt, die durch die Verbesserung der psychischen Aktivität der Kranken und eine schnelle (nach 4 bis 8 Stunden) Wiederherstellung des Bewusstseins der Kranken gekennzeichnet wurde, die in die Klinik im komatösen Zustand gelangten.

Die Elektroenzephalogramme der Untersuchung der bioelektrischen Hirnaktivität zeigten eine deutliche (von 20 bis 30 min Dauer) Verbesserung der Aktivität unter dem Auftreten des normalen alpha-Rhythmus bei der Einführung des erfindungsgemässen Arzneimittels.

Der Zustand der neuromuskulären Leitfähigkeit bei den Kranken wurde durch das Verschwinden oder die Herabsetzung der Myofibrillation nach jeder wiederholten Einführung des erfindungsgemässen Arzneimittels gekennzeichnet.

Es wurde die Erhöhung der bioelektrischen Muskelaktivität bei der willkürlichen Muskelverkürzung von 30-60 $\mu V$ auf I80-300 $\mu V$ 30 min bis I Stunde nach der Einführung des Reaktivators nachgewiesen.

Die Wirkung des erfindungsgemässen Arzneimittels auf das kardiovaskuläre System äusserte sich in der Wiederherstellung der Leitfähigkeit im zusammenziehbaren Myokard, die durch die Normalisierung des systolischen Kennwertes gekennzeichnet wurden.

Die reaktivierende Wirkung des erfindungsgemässen Arzneimittels zur Wiederherstellung der Cholinesteraseaktivität des intakten Bluts erreichte IO bis 30% von der Norm im Laufe der ersten 24 Stunden nach der Vergiftung.

Im Vergleich zu TMB-4 besitzt das erfindungsgemässe Arzneimittel eine ausgeprägte Zentralwirkung, die durch eine schnelle Wiederherstellung des Bewusstseins der Kranken und die Verbesserung der bioelektrischen Hirnaktivität gekennzeichnet wird, und übt ebenso wie TMB-4 eine deutliche periphere Wirkung aus, die sich in der Wiederherstellung der neuromuskulären Leitfähigkeit äussert.

Die Untersuchung einiger Kennziffer, die die Funktion der Leber und der Stoffwechselprozesse kennzeichnen (Einweissfraktion, Bilirubin, Transaminasenaktivität, Blutzucker, Kennwerte des Elektrolytwechsels und des Gaswechsels) zeigte, dass das erfindungsgemässe Arzneimittel zu deren Normalisierung beiträgt.

Es wird empfohlen, das erfindungsgemässe Arzneimittel intramuskulär einmalig oder wiederholt je nach der Vergiftungsschwere einzuführen.

Vor der Verwendung wird der Ampulleninhalt (0,075 g Arzneimittel)in I ml Injektionswasser aufgelöst.

Bei den anfänglichen Merkmalen der Vergiftung mit phosphororganischen Verbindungen (Aufregung, Pupillenverengung, Hyperhidrosis, Hypersalivation, anfängliche Bronchorrhoeerscheinungen) werden subkutan 2 bis 3 ml 0,I%ige Atropinsulfatlösung und intramuskulär das erfindungsgemässe Arzneimittel in einer Dosis von 0,075 g pro 60 bis 70 kg Körpermasse des Kranken eingeführt. Wenn die Symptome nicht verschwinden, werden die Arzneimittel wiederholt nach 2 bis 3 Stunden in derselben Dosierung eingeführt.

Bei schweren Vergiftungsformen werden intravenös 3 ml 0,I%ige Atropinsulfatlösung verwendet, nach 5 bis 6 min wird dieselbe Atropindosis intravenös bis zum vollen Aufhören des Bronchosphasmus und bis zur Entwicklung der Symptome der Atropinüberdosierung eingeführt. Gleichzeitig wird das erfindungsgemässe Arzneimittel in einer Dosis von 0,075 g intramuskulär in einem ein- bis dreistündigen Abstand zwischen den Injektionen im Laufe der ersten 24 Stunden Intoxikation in Kombination mit der Erhaltungstherapie mit Atropin eingeführt.

Als Kennwert der Effektivität der spezifischen Behandlung wurde eine deutliche Verbesserung der bioelektrischen Hirnaktivität (das Auftreten des normalen alpha-Rhythmus), das Aufhören der Myofibrillation und eine standhafte Erhöhung der Aktivität der Blutcholinesterase angesehen.

Die Einzeldosis des erfindungsgemässen Arzneimittels betrug 0,075 g. Die maximale Tagesdosis - 0,2 bis 0,8 g. Die Kurdosis - 0,4 bis I,6 g. Es wurden keine Nebenerscheinungen und Kontraindikationen bei der Anwendung des erfindungsgemässen Arzneimittels nachgewiesen. Das erfindungsgemässe Arzneimittel wird in einer lichtgeschützten Stelle bei einer Temperatur höchstens von +IO°C, Liste B, ausbewahrt.

## Industrielle Verwendbarkeit

Das erfindungsgemässe Antidot wird zur Behandlung akuter Vergiftungen mit phosphororganischen Verbindungen verwendet. Das erfindungsgemässe Arzneimittel wird in Kombination mit Cholinolytika (Atropin und andere) verwendet.

PATENTANSPRUCHE

I. Antidot bei der Vergiftung mit phosphororganischen Verbindungen, das einen Wirkstoff und ein pharmazeutisches Lösungsmittel vorsieht, dadurch g e k e n n z e i c h n e t, dass es als Wirkstoff N-Allyl-2-pyridinaldoximbromid folgender Formel enthält:

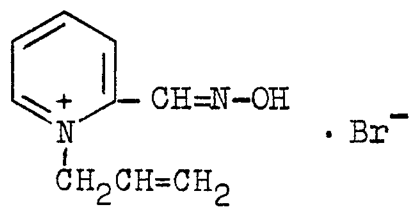

2. Antidot nach Anspruch I in Form von Injektionslösungen, dadurch g e k e n n z e i c h n e t, dass es den Wirkstoff in einer Menge von 7,5 Masse% enthält.

3. Antidot nach Anspruch I bis 2, dadurch g e k e n n z e i c h n e t, dass es als pharmazeutisches Lösungsmittel bidestilliertes Wasser enthält.

# INTERNATIONAL SEARCH REPORT

International Application No PCT/SU 88/00103

**I. CLASSIFICATION OF SUBJECT MATTER** (if several classification symbols apply, indicate all) [6]

According to International Patent Classification (IPC) or to both National Classification and IPC

$IPC^4$ A 61 K 31/44, 9/08

**II. FIELDS SEARCHED**

Minimum Documentation Searched [7]

| Classification System | Classification Symbols |
|---|---|
| $IPC^4$ | A 61 K 31/44, 9/08 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4369183 (MERCK & CO., Inc.), 18 January 1983 (18.01.83), see column 1, lines 12-18 | 1-2 |
| A | GB, A, 2016920 (DSO "PHARMACHIM"), 26 September 1979 (26.09.79), see page 1, lines 6-9 | 1-2 |
| A | US, A, 4535163 (SMITH KLINE & FRENCH LABORATORIES LIMITED), 13 August 1985 (13.08.85), see the claims<br>JP, A, 61-233616, 17.10.86<br>GB, A, 2101599, 19.01.83 | 1-2 |
| A | US, A, 4661502 (OTSUKA PHARMACEUTICAL CO., LTD), 28 April 1987 (28.04.87), see the abstract | 1-2 |
| A | GB, B, 1587076 (F. HOFFMANN-LA ROCHE & CO., AKTIENGESELLSCHAFT), 25 March 1981 (25.03.81), see page 1 & FR, A1, 2423219, 16.11.79<br>FR, B1, 2358161, 25.07.80<br>JP, B, 60-51448, 14.11.85<br>CH, A, 628811, 31.03.82 | 3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 15 November 1988 (15.11.88) | 13 January 1989 (13.01.89) |
| **International Searching Authority** | **Signature of Authorized Officer** |
| ISA/SU | |